# EUROPEAN PATENT SPECIFICATION

(11) **EP 1 887 957 B1**
(45) Date of publication and mention of the grant of the patent: **17.06.2009**
(21) Application number: 06750547.9
(22) Date of filing: 17.04.2006
(51) Int. Cl.: A61B 18/02, A61B 18/18

(54) **LEAK DETECTION SYSTEM FOR CATHETER BASED MEDICAL DEVICE**
LECKERFASSUNGSSYSTEM FÜR EINE MEDIZINISCHE VORRICHTUNG AUF KATHETERBASIS
SYSTEME DE DETECTION DE FUITE POUR DISPOSITIF MEDICAL A CATHETER

(30) Priority: 13.05.2005 US 129021
(43) Date of publication of application: 20.02.2008
(73) Proprietor: CryoCath Technologies Inc., Kirkland, Québec H9H 5H3 (CA)
(72) Inventor: LEHMANN, John, W., Wayland, Massachusetts 01778-1415 (US); AL ASMAR, Johnny, Nicosia, 1076 (CY); ABBOUD, Marwan, Pierrefonds, Québec H9K 1M7 (CA)
(74) Representative: Casey, Lindsay Joseph
(86) International application number: PCT/US2006/014540
(87) International publication number: WO 2006/124177

(56) References cited:
- US-A1- 2002 007 180
- US-A1- 2003 004 504
- US-A1- 2004 158 238
- US-A1- 2004 243 119
- US-A1- 2005 245 943

## Description

The invention relates to medical devices, and more particularly to minimally invasive surgical systems.

### BACKGROUND OF THE INVENTION

Medical devices configured for minimally invasive surgery are rapidly becoming the tools of choice for many surgical procedures. Not only do these devices provide an alternative to more invasive surgical tools and procedures, but they have also fostered the development of entirely new procedures.

Devices including highly flexible catheters, as well as rigid and semi-flexible probes have received increased attention in recent years and continue to be refined for cardiovascular, pulmonary, urogenital, and other applications. Devices for each of these applications present different technology and material challenges. Angioplasty catheters, for example, can require fluid-tight passages or channels for circulating a cooling fluid (liquid or gas) through a catheter to cool an electro-surgical structure, such as radio frequency ablation electrode, to prevent overheating of the electrode or of surrounding tissue. Similarly, a cooling or cryogenic fluid can be reduce the temperature of a structure, such as an ablation surface, to a therapeutic temperature. Some cooling fluids, however, can be harmful or fatal to the patient if they unintentionally escape from the surgical device.

Although careful fabrication techniques, quality materials, and thorough testing can reduce the chances of cooing fluid leakage, it would be desirable to provide additional system features that further minimize the occurrence of leaks; and should a leak occur, provide features that detect cooling fluid loss or escape immediately so that use of the surgical device can be terminated and patient remediation efforts can be undertaken if required.

United States Patent Specification no US-A-2004/0243119 discloses an elongated catheter device with a distal balloon assembly is adapted for endovascular insertion. Coolant injected through the device may, in different embodiments, directly cool tissue contacting the balloon, or may cool a separate internal chamber. Plural balloons may be provided, wherein a secondary outer balloon surrounds a primary inner balloon, the primary balloon being filled with coolant and acting as the cooling chamber, the secondary balloon being coupled to a vacuum return lumen to serve as a robust leak containment device and thermal insulator around the cooling chamber. One or more sensors may be disposed between the balloons or the vacuum return lumen, to detect leaks and control the flow of fluid through the device. Examples of sensors include pressure and temperature sensors, optical sensors, magnetic flow switches and flow meters.

### SUMMARY OF THE INVENTION

According to an aspect of the present invention, there is provided a medical device as specified in claim 1. According to an aspect of the present invention, there is provided a method for leak detection in a medical device as specified in claim 6.

The present invention provides a medical device having an elongate body defining an injection lumen and an exhaust lumen, as well as a first pliable element defining a cooling chamber disposed at a point along the elongate body, the cooling chamber in fluid communication with the injection lumpen and the exhaust lumpen. A second pliable element at least partially encloses the first pliable element, defining a junction between the first and second pliable element. The medical device further includes a first leak detector in fluid communication with the cooling chamber and a second leak detector in fluid communication with the junction. A check valve is included in fluid communication with the junction, the check valve further being in fluid communication with the exhaust lumen. Moreover, a cryogenic fluid supply may be in fluid communication with the injection lumen, while a vacuum source is provided in fluid communication with the exhaust lumen. A control unit is also included in communication with the first and second leak detector, wherein the control unit is responsive to output from the first and second leak detectors to control fluid flow through the medical device.

Exemplary leak detection apparatus include an impedance measurement circuit, an infrared sensor, a pulsed ultrasonic device, or a length of duplex wire having a portion of insulation removed.

### BRIEF DESCRIPTION OF THE DRAWINGS

The invention will be more fully understood from the following detailed description taken in conjunction with the accompanying drawings in which:
FIG. 1 is a schematic view of a minimally invasive surgical system including a leak detection system in accordance with the invention;
FIG. 2 illustrates an exemplary cryocatheter tip with a leak detection circuit;
FIG. 3 illustrates a porous, insulated, conductive wire within a cryocatheter tip;
FIG. 4 illustrates another leak detection device;
FIG. 5 shows an alternative embodiment of a catheter tip with a leak detector device; and
FIG. 6 illustrates an alternative embodiment of a leak detector device.

### DETAILED DESCRIPTION OF THE INVENTION

In the discussion which follows, "surgical device" is intended to encompass any surgical implement used in association with human or animal medical treatment, diagnosis, study, or analysis. More particularly, a surgical device is intended to encompass any implement or portion thereof that is entirely or partially inserted into a human or animal body by any means of entry, such as through a natural body orifice, an incision, or a puncture. The term surgical device is not intended to connote a limitation to treatment of a single body system, organ, or site. The surgical device can be rigid as a thick steel pipe, completely flexible and pliant like a thread, or have a flexibility between the two extremes. The surgical device can have a diameter that ranges from inches to microns.

As used herein, "fluid" is intended to encompass materials in a liquid state, a gas state, or in a transition state between liquid and gas, and liquid and solid. The fluid can be a "cryogenic fluid" capable of reaching or creating extremely cold temperatures well below the freezing point of water, such as below minus 20 degrees Centigrade; a "cooling fluid" that does not reach or create temperatures below the freezing point of water; a fluid capable of transferring heat away from a relatively warmer structure or body tissue; a fluid capable of transferring heat to a relatively cooler structure or body tissue; a fluid at or capable of creating a temperature between the freezing and boiling points of water; and a fluid at or capable of reaching or creating a temperature above the boiling point of water.

A "fluid path" as used herein is intended to encompass any boundary, channel or guide through which a fluid can travel. It can include concentrically disposed catheters, multi-lumen catheters, or a single loop of tubing within a sheath. The fluid path can also include connectors and valves, as well as passages in support equipment, such as the console disclosed herein.

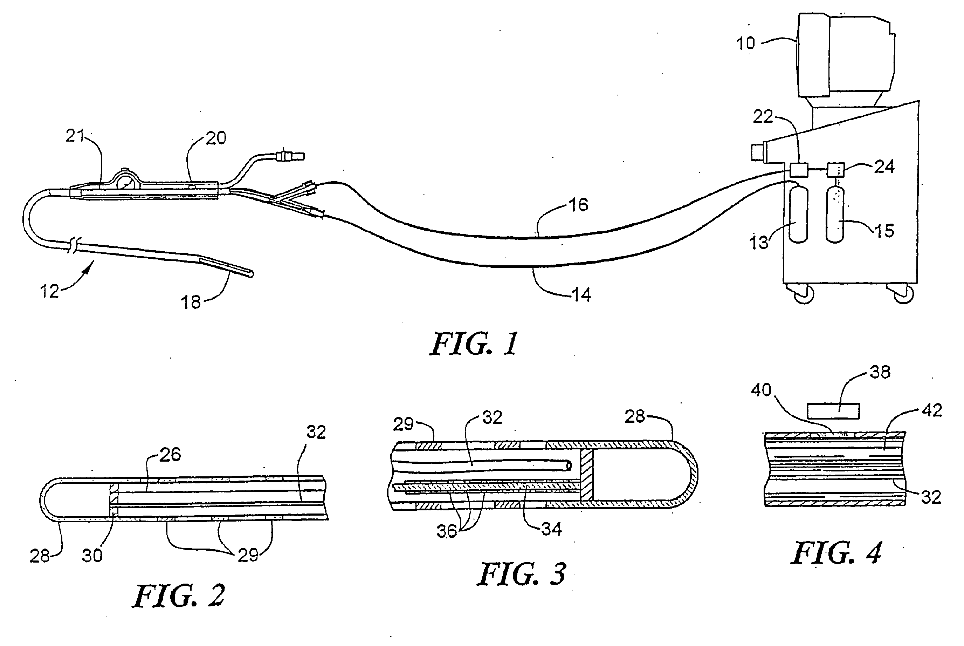

Referring now to FIG. 1, an exemplary surgical device is illustrated for minimally invasive surgery. The surgical device includes a console 10 and a multi-lumen catheter 12. The console 10 houses electronics and software for controlling and recording a surgical procedure, such as ablation, and it controls delivery of liquid refrigerant under high pressure from a supply container 13, through an umbilical 14, to the catheter 12. A second umbilical 16 is provided for transferring refrigerant from the catheter 12 to console 10. The console 10 is provided with apparatus 15 for recovery of expanded refrigerant vapor from the catheter and recompression of the vapor.

Either or both of the catheter 12 and the console 10 can be provided with detection devices that are in electrical communication with the console and which provide a signal output that can be representative of an event that indicates flow path integrity loss or a leak within a sealed catheter and/or console. As shown in FIG. 1, a first detection device or leak detector 18 can be provided in a body or tip portion of the catheter 12. A second leak detector 20 can be provided in the handle portion 21 of the catheter 12; and a third leak detector 22 can be provided in the console 10. The console 10 can be configured to respond to signal output from the leak detectors and initiate a predetermined sequence of events, such as discontinuing refrigerant injection, changing the pressure within the system, and controlling removal of refrigerant from the catheter 12.

The purpose and function of the leak detectors is better understood once another feature of the invention is introduced, namely, a vacuum pump 24, as shown in FIG. 1 in fluid communication with a catheter 12. The third leak detector 22 can be interposed between the vacuum pump 24 and the catheter 16. The vacuum pump 24 is controllable to reduce the pressure within the return lumen of the catheter 12 and the second umbilical 16 to provide a pressure ranging from a pure vacuum to a pressure just below a patient's blood pressure. For example, the vacuum can maintain a selected pressure between 80 mm Hg and 0 mm Hg. The provision of reduced pressure within the return flow path of the catheter significantly enhances patient safety because, should a leak occur, refrigerant will not squirt from the leak into the patient. Rather, bodily fluids in the treatment site will be aspirated into the catheter whereupon they are sensed by one or more of the leak detectors. In one mode of operation, when a leak is detected, the refrigerant injection is turned off automatically and vacuum is kept on to ensure that no refrigerant enters the patient's body.

Although a single type of leak detector could be functional, an exemplary embodiment of the invention is provided with three different types of leak detectors for enhanced detection probability. For example, the first leak detector 18 can be a simple circuit formed by a wire, such as a pull-wire used to help steer the catheter tip, and a conductive catheter tip portion. Specifically, as shown in FIG. 2, a wire 26 is electrically isolated from a metal catheter tip 28 and metal electrode rings 29. In the illustrated embodiment, the wire is secured to a non-conductive support element 30. Also shown is a refrigerant injection tube 32. The electrical impedance between the wire 26 and the catheter tip 28 is monitored. If a liquid enters the catheter 12 and touches the wire 26 and the tip 28, a short is created which is detectable by circuitry in the console. Alternatively, the wire 26 and one or more of the electrode rings 29 can be included in the impedance circuit.

However, some catheters 12 may include multiple conductors running within one or more lumens and electrical insulation on the conductors is necessary to avoid unwanted electrical connections and interferences. Many such catheters also contain uninsulated wires, for example as mechanical deflectors to alter catheter configuration, or for example as stiffening agents to alter catheter flexibility or pushability. However, if the pull wire (or other wire that is part of the leak detection circuit) contacts another uninsulated wire, electrode ring or other conductive element, a false leak detection signal could be generated. Accordingly, a form of insulation that provides mechanical insulation while allowing fluid conductivity is desirable.

FIG. 3 discloses a wire 34 (such as a pull wire) that is part of the leak detection circuit. The wire 34 is covered with a porous material 36, such as a fabric, salt-depleted polymer, or laser drilled polymer, that provides mechanical insulation in the dry state by the physical bulk and separation of the porous material, which allows passage of ionic fluids to the thus insulated wire to complete the electrical leak detection circuit.

Although the first leak detector 18 is well suited for detecting leaks at or near the distal end of the catheter 12, a leak may develop between the distal end and the handle portion 21 of the catheter and an infrared sensor can be disposed in the handle as the second leak detector 20. As soon as the first and/or second leak detectors output a signal to the console indicative of a leak, the refrigerant injection can be stopped. In an exemplary embodiment, shown in FIG. 4, an infrared sensor 38 with a wavelength sensitive to blood composition is disposed in sensing range with a transparent window 40 or tube along or forming part of the return fluid flow path 42.

Even though refrigerant injection is stopped, it can still be desirable to apply vacuum to the catheter to withdraw refrigerant already introduced into the catheter, along with refrigerant contaminated blood. Thus, a third leak detector 22 (shown in FIG. 1) is provided further downstream in the fluid flow path to not only provide a last opportunity for detection, but to also detect when a selected volume of blood has been aspirated (a relatively small amount) and to then terminate vacuum operation or aspiration. Depending on placement of the third leak detector, it can prevent blood contamination of the entire fluid flow path within the console 10.

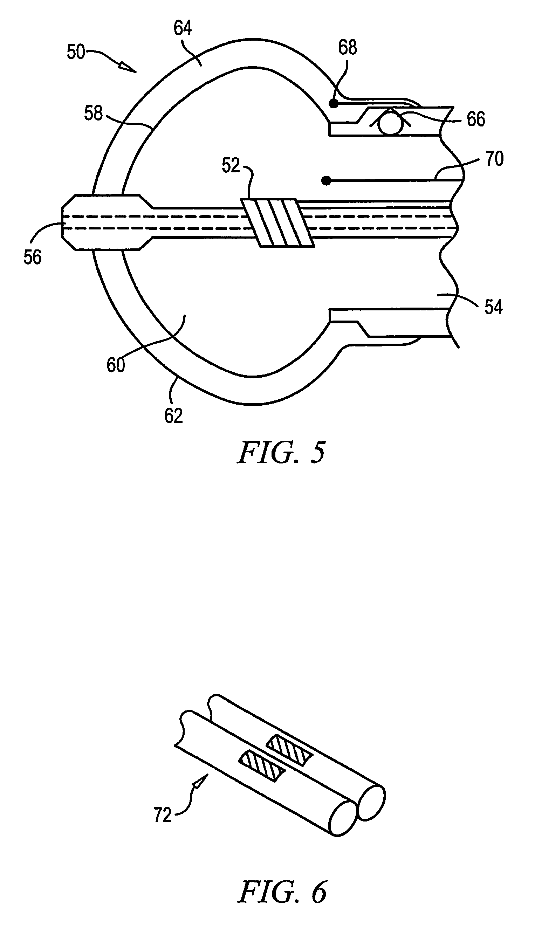

In an alternative embodiment, leak detection may be provided for a catheter having one or more expandable elements, e.g., a balloon catheter or the like. FIG. 5 shows an alternative body or tip portion 50 of the catheter 12. The multi-lumen catheter 12 defines both an injection lumen 52 and an exhaust lumen 54. A guidewire lumen 56 is also provided, such that a portion of the catheter may be positionable over a guidewire to aid in steering the catheter to a desired tissue site. Although FIG. 5 shows the injection lumen 52 coiled around a portion of the guidewire lumen 56, the injection lumen 52 may be any conduit situated such that it is capable of delivering fluid to the cooling chamber 60. The catheter further includes a first pliable element 58 defining a cooling chamber 60 disposed along a portion of the catheter, where the cooling chamber 60 is in fluid communication with both the injection and exhaust lumens. The injection lumen 52, cooling chamber 60, and exhaust lumen 54 define a first fluid path through which a cryogenic fluid or the like may circulate.

The catheter 12 further provides a second pliable element 62 at least partially enclosing the first pliable element 58, thereby defining a junction 64 between the first and second pliable elements. The second pliable element 62 provides a safeguard to prevent fluid from leaking out of the cooling chamber 60 and into surrounding tissue should the first pliable element 58, and therefore the cooling chamber 60, rupture or develop a leak. The junction 64 between the first and second pliable elements may be substantially under a vacuum, such that the first and second pliable elements are generally in contact with each other, with little or no open space between them.

A check valve 66 is provided in fluid communication with the junction 64 between the first and second pliable elements, with the check valve 66 also being in fluid communication with the exhaust lumen 54. The check valve 66 is a one way valve that prevents fluid from traveling from the exhaust lumen 54 into the junction 64 between the first and second pliable elements, yet allows fluid, if any, to flow from the junction 64 between the first and second pliable elements towards the exhaust lumen 54. The check valve 66 may be such that the valve opens automatically in response to a pressure change in the junction 64.

A first leak detector 68 may be included in fluid communication with the junction 64 to provide the ability to detect any ingress of blood or fluid into the junction 64, thereby indicating a leak or other structural compromise of the catheter. Further, a second leak detector 70 may be included in fluid communication with the exhaust lumen 54, which could indicate when a leak in the guidewire lumen or other structural breach has allowed fluid ingress into the exhaust lumen 54. Although the first and second leak detectors are described as independent, they may be in communication with each other at some point along the length of the catheter, i.e., the second leak detector 70 may be an extension or branch of the first leak detector 68. The first and second leak detectors can detect an ingress of fluid by providing an impedance measurement, which would change upon the presence of blood or other foreign fluids within the junction 64 or exhaust lumen 54. Alternatively, the leak detectors may include an insulated length of duplex wire 72, where a portion of the wire insulation has been stripped as shown in FIG. 6. Although the individual wires remain insulated from each other even after being stripped, a short between the wires will be created by the presence of a conductive fluid, thereby indicating a leak. The leak detectors may be in electrical communication with the console 10 and can provide a signal output representative of a loss of flow path integrity. Subsequently, the console 10 can initiate a predetermined sequence of events, such as discontinuing fluid injection, or evacuation of the fluid remaining in the catheter.

In an exemplary operation of the embodiment described above, fluid flow is provided through the first fluid path. At least partially surrounding the first pliable element 58 is the second pliable element 62, with the junction 64 formed therebetween substantially under a vacuum. As the check valve 66 is provided in fluid communication with both the junction 64 between the first and second pliable element as well as the exhaust lumen 54, the fluid pressure in the exhaust lumen 54 is higher than that of the vacuum pressure in the junction 64. As a result, the check valve 66 remains closed under normal operating conditions, preventing any fluid flow through the check valve 66.

However, in the event of a leak or rupture of either the first pliable element 58 or the second pliable element 62, fluid will flow into the junction 64 between the two pliable elements, thus eliminating the vacuum in the junction 64. As a result, if the pressure in the junction 64 exceeds that of the pressure in the exhaust lumen 54 downstream of the check valve 66, then the check valve 66 will open. Subsequently, as the check valve 66 is forced open due to the pressure change, a second fluid path results, which flows from the cooling chamber 60 into the junction 64 between the first and second pliable element 62, through the check valve 66, and into the exhaust lumen 54.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope of the invention, which is limited only by the following claims.

## Claims

1. A medical device comprising:
an elongate body defining an injection lumen (52) and an exhaust lumen (54);
a first pliable element (58) defining a cooling chamber (60) disposed at a point along the elongate body, the cooling chamber in fluid communication with the injection lumen and the exhaust lumen;
a second pliable element (62) at least partially enclosing the first pliable element, defining a junction (64) between the first and second pliable element;
a first leak detector (70) in fluid communication with the cooling chamber; and
a second leak detector (60) in fluid communication with the junction
**characterized in that** said medical device further comprises a check valve in fluid communication with the junction, the check valve further in fluid communication with the exhaust lumen.

2. The medical device according to Claim 1, further comprising a supply of cryogenic fluid in fluid communication with the injection lumen.

3. The medical device according to Claim 2, further comprising a vacuum source in fluid communication with the exhaust lumen.

4. The medical device according to Claim 3, further comprising a control unit in communication with the first and second leak detector, wherein the control unit is responsive to output from the first and second leak detectors to control fluid flow through the medical device.

5. The medical device according to claim 1 wherein the first leak detector includes a length of insulated duplex wire having a portion of the insulation removed; and wherein the second leak detector includes a length of insulated duplex wire having a portion of the insulation removed.

6. A method for leak detection in a medical device, comprising:
providing a medical device having an elongate body defining an injection lumen (52) and an exhaust lumen, a first pliable element (58) defining a cooling chamber (66) disposed at a point along the elongate body, the cooling chamber (60) in fluid communication with the injection lumen and the exhaust lumen, a second pliable element (62) at least partially enclosing the first pliable element, defining a junction between the first and second pliable element, a check valve in fluid communication with the junction, the check valve further in fluid communication with the exhaust lumen; a first leak detector (70) in fluid communication with the cooling chamber; and a second leak detector (68) in fluid communication with the junction;
providing a control unit in communication with the first and second leak detectors, the control unit able to modify fluid flow through the medical device; and
discontinuing fluid flow of fluid in response to an output from the first and second leak detectors.

7. The method according to Claim 6, further comprising the step of evacuating fluid from the medical device.

## Patentansprüche

1. Medizinische Einrichtung, die Folgendes umfasst:
einen länglichen Körper, der ein Injektionslumen (52) und ein Auslasslumen begrenzt (54);
ein erstes biegsames Element (58), das eine an einem Punkt entlang des länglichen Körpers angeordnete Kühlkammer (60) begrenzt, wobei die Kühlkammer in Fluidkommunikation mit dem Injektionslumen und dem Auslasslumen steht;
ein zweites biegsames Element (62), das zumindest teilweise das erste biegsame Element umschließt, wobei ein Übergang (64) zwischen dem ersten und zweiten biegsamen Element gebildet wird;
einen ersten Leckagedetektor (70) in Fluidkommunikation mit der Kühlkammer; und
einen zweiten Leckagedetektor (68) in Fluidkommunikation mit dem Übergang;
**dadurch gekennzeichnet, dass** die medizinische Einrichtung weiter ein Sperrventil in Fluidkommunikation mit dem Übergang umfasst, wobei das Sperrventil weiter in Fluidkommunikation mit dem Auslasslumen steht.

2. Medizinische Einrichtung nach Anspruch 1, die weiter einen Vorrat von kryogenem Fluid in Fluidkommunikation mit dem Injektionslumen aufweist.

3. Medizinische Einrichtung nach Anspruch 2, die weiter eine Vakuumquelle in Fluidkommunikation mit dem Auslasslumen aufweist.

4. Medizinische Einrichtung nach Anspruch 3, die weiter eine Steuereinheit in Kommunikation mit dem ersten und zweiten Leckagedetektor aufweist, wobei die Steuereinheit auf Ausgaben von dem ersten und zweiten Leckagedetektor reagiert, um Fluidfluss durch die medizinische Einrichtung zu steuern.

5. Medizinische Einrichtung nach Anspruch 1, bei der der erste Leckagedetektor eine Länge von isoliertem Duplexkabel enthält, bei dem ein Teil der Isolierung entfernt ist; und wobei der zweite Leckagedetektor eine Länge von isoliertem Duplexkabel enthält, bei dem ein Teil der Isolierung entfernt ist.

6. Verfahren zur Leckageermittlung in einer medizinischen Einrichtung, das folgende Schritte umfasst:
Bereitstellen einer medizinischen Einrichtung, die einen länglichen Körper, der ein Injektionslumen (52) und ein Auslasslumen begrenzt, ein erstes biegsames Element (58), das eine an einem Punkt entlang des länglichen Körpers angeordnete Kühlkammer (66) begrenzt, wobei die Kühlkammer (60) in Fluidkommunikation mit dem Injektionslumen und dem Auslasslumen steht, ein zweites biegsames Element (62), das das erste biegsame Element wenigstens teilweise umschließt, wobei ein Übergang zwischen dem ersten und dem zweiten biegsamen Element gebildet wird, ein Sperrventil in Fluidkommunikation mit dem Übergang, wobei das Sperrventil weiter in Fluidkommunikation mit dem Auslasslumen steht; einen ersten Leckagedetektor (70) in Fluidkommunikation mit der Kühlkammer; und einen zweiten Leckagedetektor (68) in Fluidkommunikation mit dem Übergang umfasst;
Bereitstellen einer Steuereinheit in Kommunikation mit dem ersten und zweiten Leckagedetektor, wobei die Steuereinheit Fluidfluss durch die medizinische Einrichtung modifizieren kann; und
Abbrechen des Fluidflusses als Reaktion auf eine Ausgabe von dem ersten und zweiten Leckagedetektor.

7. Verfahren nach Anspruch 6, das weiter den Schritt aufweist, Fluid aus der medizinischen Einrichtung zu evakuieren.

## Revendications

1. Dispositif médical comportant :
un corps allongé définissant une lumière d'injection (52) et une lumière de sortie (54) ;
un premier élément souple (58) définissant une chambre de refroidissement (60) se trouvant en un point le long du corps allongé, la chambre de refroidissement étant en communication fluide avec la lumière d'injection et avec la lumière de sortie ;
un deuxième élément souple (62) renfermant au moins partiellement le premier élément souple, définissant une jonction entre le premier élément souple et le deuxième élément souple ;
un premier détecteur de fuite (70) en communication fluide avec la chambre de refroidissement ; et
un deuxième détecteur de fuite (68) en communication fluide avec la jonction ;
**caractérisé en ce que** ledit dispositif médical comporte par ailleurs un clapet de non-retour en communication fluide avec la jonction, le clapet de non-retour étant par ailleurs en communication fluide avec la lumière de sortie.

2. Dispositif médical selon la revendication 1, comportant par ailleurs une alimentation de fluide cryogénique en communication fluide avec la lumière d'injection.

3. Dispositif médical selon la revendication 2, comportant par ailleurs une source de vide en communication fluide avec la lumière de sortie.

4. Dispositif médical selon la revendication 3, comportant par ailleurs un module de commande en communication avec le premier détecteur de fuite et avec le deuxième détecteur de fuite, dans lequel le module de commande réagit à toute sortie en provenance du premier détecteur de fuite et du deuxième détecteur de fuite à des fins de commande de l'écoulement de fluide au travers du dispositif médical.

5. Dispositif médical selon la revendication 1, dans lequel le premier détecteur de fuite comprend une longueur de fil double isolé dont une portion de l'isolement a été retirée ; et dans lequel le deuxième détecteur de fuite comprend une longueur de fil double isolé dont une portion de l'isolement a été retirée.

6. Procédé de détection de fuite dans un dispositif médical, comportant :
la mise en oeuvre d'un dispositif médical ayant un corps allongé définissant une lumière d'injection (52) et une lumière de sortie, un premier élément souple (58) définissant une chambre de refroidissement (66) se trouvant en un point le long du corps allongé, la chambre de refroidissement (60) étant en communication fluide avec la lumière d'injection et avec la lumière de sortie, un deuxième élément souple (62) renfermant au moins partiellement le premier élément souple, définissant une jonction entre le premier élément souple et le deuxième élément souple, un clapet de non-retour en communication fluide avec la jonction, le clapet de non-retour étant par ailleurs en communication fluide avec la lumière de sortie ; un premier détecteur de fuite (70) en communication fluide avec la chambre de refroidissement ; et un deuxième détecteur de fuite (68) en communication fluide avec la jonction ;
la mise en oeuvre d'un module de commande en communication avec le premier détecteur de fuite et avec le deuxième détecteur de fuite, le module de commande étant en mesure de modifier l'écoulement de fluide au travers du dispositif médical ; et
l'interruption de l'écoulement du fluide en réaction à une sortie en provenance du premier détecteur de fuite et du deuxième détecteur de fuite.

7. Procédé selon la revendication 6, comportant par ailleurs l'étape consistant à évacuer du fluide du dispositif médical.
